# EUROPEAN PATENT SPECIFICATION

(11) **EP 2 240 087 B1**
(45) Date of publication and mention of the grant of the patent: **24.07.2019**
(21) Application number: 08713701.4
(22) Date of filing: 10.01.2008
(51) Int. Cl.: A61B 17/06, A61B 17/00, C22C 27/04

(54) **TUNGSTEN ALLOY SUTURE NEEDLES**
NÄHNADELN AUS EINER WOLFRAM-LEGIERUNG
AIGUILLES EN ALLIAGE DE TUNGSTÈNE POUR SUTURE

(43) Date of publication of application: 20.10.2010
(73) Proprietor: ETHICON, INC., Somerville, New Jersey 08876 (US)
(72) Inventor: MAURER, Robert, E., Belle Mead, New Jersey 08502 (US); CICHOCKI, Frank, R., Jr., Easton Pennsylvania 18045 (US); REYNOLDS, Eugene, D., Avon-by-the-Sea New Jersey 07717 (US)
(74) Representative: Kirsch, Susan Edith
(86) International application number: PCT/US2008/050733
(87) International publication number: WO 2009/088514

(56) References cited:
- EP-A2- 0 893 695
- EP-B1- 0 646 352
- US-A- 3 236 699
- US-A- 3 637 374
- US-A1- 2003 074 022

## Description

### FIELD OF THE INVENTION

The present invention relates to curved suture needles, and in particular to curved tungsten alloy suture needles having a desirable combination of stiffness, strength and ductility. More specifically, the present invention relates to heat treated curved tungsten alloy suture needles that exhibit superior bending stiffness properties. The scope of protection of the invention is defined in appended claims 1, 11 and 13. Preferred embodiments are disclosed in the dependent claims.

### BACKGROUND OF THE INVENTION

Certain surgeries, particularly coronary artery bypass surgery, necessarily involve the use of suture needles of small diameter having exceedingly high bending stiffness and strength. In particular, surgery of this type requires that the suture needle's path be closely controlled. If the needle flexes excessively as it enters the tissue or as it pierces the inner surface of e.g., a blood vessel before re-emerging, improper placement of the needle and serious trauma to the tissue and the patient can occur. In use, suture needles are subjected to substantial stressing forces, since the force used to drive the needle into and through tissue (e.g., a blood vessel and the like) needs to be sufficient to overcome frictional drag through the tissue. These forces resisting needle penetration are commonly exacerbated in patients undergoing cardiovascular surgery, who exhibit calcified or toughened tissue due to coronary artery disease. In these procedures, the suture needle must be able to pass through not only the blood vessel, but also any hard calcified tissue that may be located along the periphery of the blood vessel lumen. A compliant needle will deflect elastically during tissue penetration resulting in a loss of placement control. As such, it is preferable that the needle should have a relatively high bending stiffness, that is, a low tendency to flex and high tendency to retain its configuration when subjected to a deforming force. Hence, stiffness in bending is an essential property for the handling and performance of suture needles. A stiff needle resists elastic deflection and can thus be directed as intended to provide a high level of control.

ASTM standard F1840-98a (Reapproved 2004) provides standard terminology for surgical suture needles and ASTM standard F1874-98 (Reapproved 2004) provides details of a standard test method for bend testing of needles used in surgical sutures. Two different measures for the strength of surgical suture needles are used, namely, yield bend moment, which is the amount of moment required to initiate plastic deformation during a bend test, and maximum bend moment, which is the greatest moment applied to a needle during a bend test. This later value of maximum bend moment is typically measured at a point where the needle has undergone substantial plastic deformation and is generally higher than the yield bend moment or point at which plastic deformation initiates. The point of deflection at which plastic deformation initiates, or more formally according to ASTM standards, the angle at which the yield bend moment occurs, is referred to as the yield bend angle.

Both needle bending strength and needle bending stiffness influence handling characteristics, as well as penetration performance and efficacy of the suture needle. It is important to note that in almost all circumstances, the suture needle should be used in applications where the yield bend moment is not exceeded, since above this value, the needle will bend plastically, losing its original shape, and will no longer function as intended. It is thus apparent that a desirable characteristic of a suture needle is a high yield bend moment, which is a manifestation of the bending strength of the suture needle. Below the yield bend moment, the resistance of bending of the suture needle is best characterized by the needle bending stiffness. Needle bending stiffness is a critical measure of the resistance to elastic, or recoverable bending of the suture needle before needle deflection reaches the yield bend angle and can be calculated as the yield bend moment divided by the yield bend angle. If a straight or curved suture needle has a low value of bending stiffness, substantial bending of the needle will occur for a given bend moment, whereas if a straight or curved suture needle exhibits a high bending stiffness value, relatively little elastic bending of the needle will occur for a given bend moment. Surgeons will tend to perceive a high degree of elastic bending as a loss of control or as a poor penetration performance since the needle point is not translating directly with the motion of their hands. As such, needle bending stiffness may be recognized as a quintessential measure of needle performance in most surgical applications.

Hence, the desirable bend properties for a suture needle are high bending stiffness, as well as bending strength manifested as high yield bend moment and ductility, in order to penetrate tissue which is being sutured without undue flexing, plastic bending, or breaking during a surgical procedure.

The needle should also not be brittle; if any portion of the needle is too brittle it may break during use if too much force is applied. The needle should instead be ductile, which is the ability to bend without breaking. Curved suture needles are commonly bent through a bend angle of 90 degrees and then manually reshaped to their original curvature to assess ductility. Those skilled in the art of needle making will recognize this procedure as the reshaping process and will further recognize that the higher the number of reshape processes that a needle can withstand without breaking the more ductile it is.

USP 5,415,707 describes tungsten alloy surgical needles that exhibit high tensile yield strength in excess of 1.724 GPa (250000 psi), high tensile modulus of elasticity or stiffness in excess of 310.3 GPa (45 x 10⁶ psi), and high ductility. The needles described therein preferably comprise about 3 to about 6 weight percent of rhenium, rhodium and/or iridium. Data presented in USP 5,415,707 was derived from straight uncurved needles.

As described in USP 5,415,707, tungsten alloys have exceptionally high stiffness along with other desirable physical properties. Tungsten alloys derive their strength from their high dislocation density and the natural resistance to deformation that occurs via dislocation-dislocation interaction as a stress is applied. However, the exceptionally high stiffness of such tungsten alloys in wire and straight needle form does not necessarily translate to high bending stiffness when such alloys are used to make curved suture needles, since the curving process during needle manufacture imparts stresses that act to reduce the bending stiffness of the curved suture needle. It is believed that during the curving portion of the needle manufacture process, the dislocations in the tungsten alloy move to high energy locations within the microstructure, or locations where high strain field exist locally around the dislocations. When a moderate unbending force is applied to the curved suture needle, the dislocations in the high energy locations readily slip to positions of lower energy or lower local strain. The slip of these dislocations to lower energy positions manifests itself as limited plastic deformation, resulting in a relatively low stiffness in bending or low yield bend moment.

Prior art EP0646352 B1 discloses selection of tungsten alloys for the forming of curved surgical needles. Prior art US 2003/074022 A1 also discloses formation of curved suture needles; specifically forming of said needles using plasma polymerization process to apply a silicone coating to produce siliconized needle with reduced tissue penetrating force after a number of passages through tissue.

There is a need for tungsten alloy suture needles that exhibit high bending strength and high bending stiffness, particularly when the suture needle is a curved needle.

### SUMMARY OF THE INVENTION

It has now been discovered that suture needles having a desirable combination of stiffness, strength and ductility can be formed from a tungsten alloy, by a method comprising the steps of (1) forming needle blanks comprising a tungsten alloy into a suture needle; and (2) heating said suture needle to a temperature ranging from 350°C to below the recrystallization temperature of the alloy.

### DESCRIPTION OF THE DRAWINGS

FIG. 1 is a graph comparing the bending performance of an 0.2032 mm (.008") diameter curved suture needle produced from a tungsten 26% rhenium alloy to an equivalent suture needle produced from a curved 4310 stainless steel alloy.
FIG. 2 is a graph showing the bending performance of tungsten 25.75% rhenium suture needles as a function of heat treatment temperature for a duration of 0.5 hour.

### DETAILED DESCRIPTION OF THE INVENTION

The suture needles of the present invention are formed from an alloy of tungsten. The tungsten alloy may comprise one or more metals selected from the group consisting of rhenium, osmium, tantalum, or molybdenum. Preferably, the alloy is a tungsten-rhenium alloy, and has no more than trace amounts of other elements present. The metal other than tungsten may be present in an amount up to about 30 weight percent of the alloy, and more preferably is present in an amount ranging from about 20 to about 26 weight percent of the alloy.

The suture needle preferably has a diameter effective to permit satisfactory usage in fine surgery. Typically, the diameter will be less than about 1.524 mm (60 mils) (thousandths of an inch), preferably less than about 0.381 mm (15 mils), down to about 0.0254 mm (1 mil), and preferably about 0.03556 mm to about 0.3048 mm (1.4 to about 12 mils). It will be recognized that the suture needle may have a circular body cross-section, and that the needle may also be of a non-circular cross-sectional shape such as triangular; trapezoidal; rectangular; hexagonal; elliptical; or rectangular wherein the opposed shorter ends of the rectangle are rounded into semicircles. By "diameter" herein is meant the square root of (4A/π) where A is the cross-sectional area. The needle may be provided with a "ribbon" shape with a single set of opposing flat sides, or a rectangular or "I-beam" shape, or with a cross-section which smoothly undergoes transition from the point to a circular cross-section, to a rectangular cross-section having rounded and then sharper corners, as described in U.S. Pat. No. 4,799,484.

The improvement in bending strength and stiffness is especially advantageous for curved needles. Preferably, the needle is curved through a radius of curvature, which need not be constant but is preferably constant. Thus, more preferred shapes of the needles of the present invention comprise sections of a circle, such as a quarter circle, three-eighths circle, half circle, or five-eighths of a circle.

Following the final drawing of the tungsten alloy wire to the final desired diameter, one end of the needle is given a point having the desired shape, the point being provided by any conventional technique such as grinding. Optionally, the body may be formed by pressing or grinding operations into the variety of shapes. The needle may then be given its desired curvature, typically by rolling around a mandrel of the desired radius of curvature. The opposite end of the needle is given an opening in its end, or other means by which the end of a suture can be attached to the needle by swaging or the like.

In order to impart improved bending strength and stiffness to the suture needle described herein, after a curvature has been imparted to the needle, the curved needle is heated to a temperature below the recrystallization temperature of the tungsten alloy. It shall be noted that for purposes of this disclosure, recrystallization temperature is defined as any temperature in which the microstructure of the tungsten alloy suture needles may be changed via the formation of new grains. Preferably, the suture needle is heated to a temperature ranging from about 700 to about 1900 °C. In one embodiment of the invention, the suture needle is heated to a temperature ranging from about 800 to about 1150 °C in an inert or reducing atmosphere for about 0.5 hours to impart bending stiffness to the surgical needle. Needles may also be attached to a tape or other conveyer material and passed transiently in the vicinity of a heat source. In this way the exposure time to elevated temperature would be limited, since it will be recognized that higher temperatures for shorter periods of time are effective to achieve the desired stiffening effect. Examples of an inert or reducing atmosphere include, but are not limited to, vacuum, argon gas, nitrogen gas, hydrogen gas, or gas mixtures thereof.

In an alternate embodiment, the suture needle is heated to a temperature ranging from about 350 to about 900 °C in an oxidizing atmosphere, in order to impart a robust adherent black, blue, or yellow oxide surface coating to the tungsten alloy suture needle described herein. For example, the suture needles and/or the needle blanks may be placed flat on a setter plate and introduced into a preheated furnace at a temperature between 350 and about 900 °C. Alternatively, the needles may be placed in the furnace at room temperature as the furnace ramps up to the target temperature and then back down to room temperature. Needles may also be attached to a tape or other conveyer material and passed transiently in the vicinity of a heat source. Exposure time may range from seconds to several hours, depending upon the temperature. More preferably temperatures range from about 400 and about 600 °C for duration of about 0.25 to about 1 hour. Examples of an oxidizing atmosphere include, but are not limited to, oxygen-rich atmosphere, air, or a carbon dioxide / carbon monoxide gas mixture that decomposes or reacts with the tungsten alloy surface to form an oxide.

In another embodiment, the suture needle may first be heated to a temperature ranging from about 700 to about 1900°C in an inert or reducing atmosphere, followed by heating to a temperature ranging from about 350 to about 900 °C in an oxidizing atmosphere, to impart improved bending stiffness and a robust adherent black, blue, or yellow surface coating to the tungsten alloy suture needles.

The needle may also be provided with a coating, for instance, a polymeric coating, in accordance with known techniques, if desired. The needle is then attached to the suture, packaged and sterilized, again in accordance with conventional techniques.

The suture needles of the present invention are characterized by a desirable combination of bending stiffness, strength and ductility. For the needles of the present invention, the wire tensile yield strength is generally at least about 1.724 GPa (250000 psi). A high wire tensile yield strength is useful as it indicates the ability of the needles of the present invention to withstand potentially deforming stresses without suffering permanent deformation.

The wire from which the needles of the present invention are made also exhibits uniquely high Young's modulus of elasticity, generally at least about 400 GPa. The high Young's modulus is desirable in that it reflects the potential for higher stiffness and the ability of the needles of the present invention to withstand potentially deforming stresses by retaining their shape, without undue flexing. However, in practice, as described above, a high Young's modulus of the wire alone does not directly translate into a high bending stiffness for a curved suture needle. Indeed to capitalize on the intrinsic material stiffness, a heat treatment is applied to the curved suture needles, as described above.

The properties of the suture needles of the present invention are illustrated in the following examples, which are provided for purposes of illustration and should not be interpreted as limiting in any way the scope of the claims appended hereto.

### EXAMPLE 1

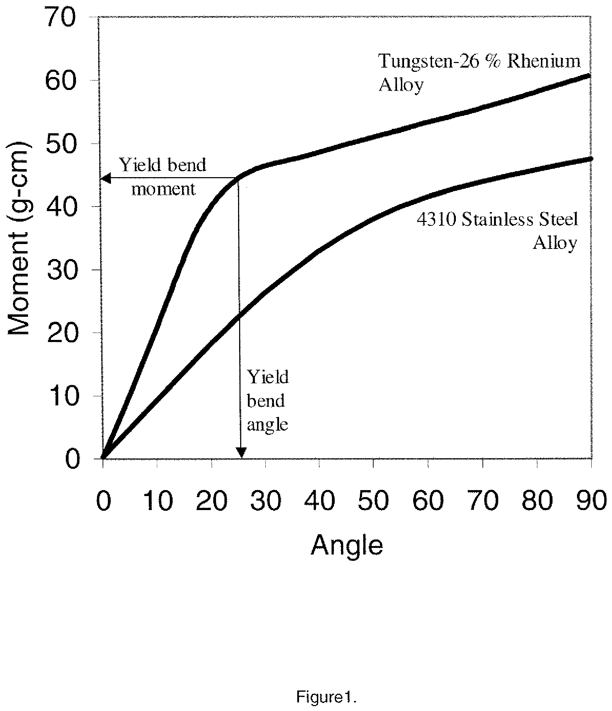

A graph comparing the bending performance of a heat treated curved 0.2032 mm (.008") diameter suture needle produced from a tungsten 26% rhenium alloy to an equivalent curved suture needle produced from a commercial 4310 stainless steel alloy used in the manufacture of suture needles is provided in Fig. 1. All tests were conducted according to ASTM standard F1874-98. The yield bend moment and yield bend angle are marked on the graph. The slope of the tungsten-rhenium alloy suture needle up to the yield bend moment represents bending stiffness and is markedly greater than that provided by the equivalent 4310 stainless steel alloy. The heat treatment applied to the tungsten alloy suture needle was conducted under an argon 2%hydrogen atmosphere at 1000 °C for 0.5 hrs.

### Example 2

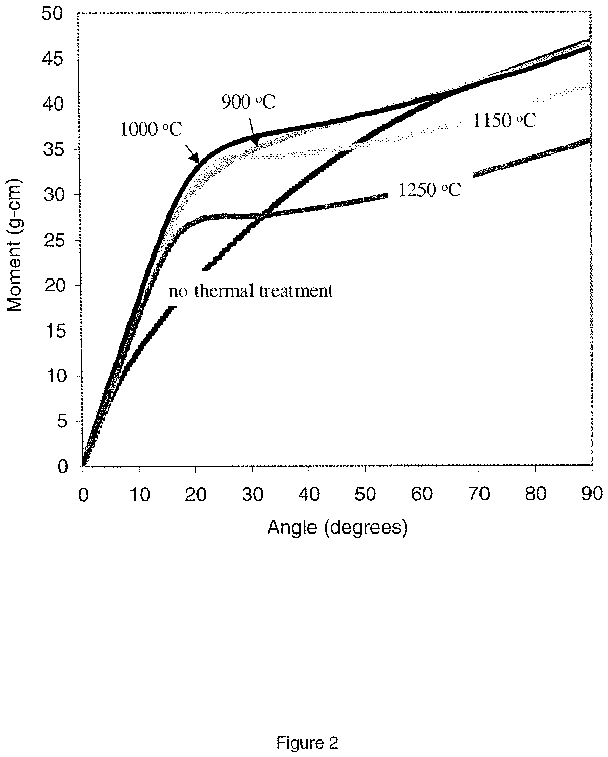

A graph comparing the bending performance of curved 0.2032 mm (.008") diameter suture needles produced from a tungsten 25.75% rhenium alloy after thermal treatment for 0.5 hr over a range of temperature is shown in Figure2.
Heat treatment was conducted under argon 2% hydrogen gas to maintain an inert non-oxidizing atmosphere. All tests were conducted according to ASTM standard F1874-98. A marked increase in bending stiffness occurs with the application of heat treatment. A maximum in bending stiffness is attained with a thermal treatment of 1000 °C for 0.5 hr. At temperatures above and below 1000 °C a decrease in the yield bend moment occurs.

It shall be recognized that similar results may be achieved with shorter duration thermal treatments at elevated temperatures and result in an upward shift for the optimal heat treatment temperature. Likewise, extended duration thermal treatments at lower temperatures may also be effective and result in a downward shift of the optimal treatment temperature.

## Claims

1. A method for making a curved tungsten alloy suture needle comprising the step of heating curved tungsten alloy needle blanks or a curved tungsten alloy suture needle to a temperature ranging from 350°C to below the recrystallization temperature of the alloy.

2. The method of claim 1, wherein the tungsten alloy needle blanks or needle is heated to a temperature ranging from 350 to 1900 °C.

3. The method of claim 2, wherein the tungsten alloy needle blanks or needle comprises at least one or more metals selected from the group consisting of rhenium, tantalum or molybdenum.

4. The method of claim 3, wherein the tungsten alloy needle blanks or needle comprises rhenium.

5. The method of claim 2, wherein the tungsten alloy needle blanks or needle comprises up to 30 weight percent rhenium, and the balance tungsten.

6. The method of claim 5, wherein the tungsten alloy needle blanks or needle comprises 20 to 26 weight percent rhenium, and the balance tungsten.

7. The method of claim 6, wherein the tungsten alloy needle blanks or needle is heated for 0.5 hours between 800 to 1150 °C.

8. The method of claim 1, wherein the curved suture needle is heated to a temperature ranging from 700 to 1900 °C in an inert or reducing atmosphere.

9. The method of claim 3, wherein the tungsten alloy needle blanks or needle is heated to a temperature ranging from 800 to 1150 °C in an inert or reducing atmosphere.

10. The method of claim 3, wherein the tungsten alloy needle blanks or needle is heated to a temperature ranging from 350 to 900 °C in an oxidizing atmosphere.

11. A curved suture needle obtained by the process of claim 1, wherein said process comprises (1) forming needle blanks comprising up to 30 weight percent rhenium, and the balance tungsten, into a curved suture needle; and (2) heating said curved suture needle to a temperature ranging from 700 to 1900 °C in an inert or reducing atmosphere for 0.01 to 1 hour.

12. The suture needle of claim 11, wherein the process further comprises the step of heating said needle to a temperature ranging from 350 to 900 °C in an oxidizing atmosphere for 0.01 to 1 hour.

13. A curved suture needle obtained by the process of claim 1, wherein said process comprises heating curved tungsten alloy surgical blanks or a curved needle to a temperature ranging from 350 to 900 °C in an oxidizing atmosphere for 0.01 to 1 hour.

## Patentansprüche

1. Verfahren zum Herstellen einer gebogenen Nähnadel aus einer Wolframlegierung, das den Schritt des Erwärmens gebogener Nadelrohlinge aus einer Wolframlegierung oder von einer gebogenen Nähnadel aus einer Wolframlegierung auf eine Temperatur umfasst, die zwischen 350 °C und unterhalb der Rekristallisationstemperatur der Legierung liegt.

2. Verfahren nach Anspruch 1, wobei die Nadelrohlinge oder Nadel aus einer Wolframlegierung auf eine Temperatur zwischen 350 und 1900 °C erwärmt werden bzw. wird.

3. Verfahren nach Anspruch 2, wobei die Nadelrohlinge oder Nadel aus einer Wolframlegierung mindestens ein oder mehrere Metalle umfasst, die aus der Gruppe bestehend aus Rhenium, Tantal oder Molybdän ausgewählt sind.

4. Verfahren nach Anspruch 3, wobei die Nadelrohlinge oder Nadel aus einer Wolframlegierung Rhenium umfasst bzw. umfassen.

5. Verfahren nach Anspruch 2, wobei die Nadelrohlinge oder Nadel aus einer Wolframlegierung bis zu 30 Gewichtsprozent Rhenium und den Rest bis 100 Gewichtsprozent Wolfram umfasst bzw. umfassen.

6. Verfahren nach Anspruch 5, wobei die Nadelrohlinge oder Nadel aus einer Wolframlegierung 20 bis 26 Gewichtsprozent Rhenium und den Rest bis 100 Gewichtsprozent Wolfram umfasst bzw. umfassen.

7. Verfahren nach Anspruch 6, wobei die Nadelrohlinge oder Nadel aus einer Wolframlegierung 0,5 Stunden lang zwischen 800 und 1150 °C erwärmt wird bzw. werden.

8. Verfahren nach Anspruch 1, wobei die gebogene Nähnadel auf eine Temperatur zwischen 700 und 1900 °C in einer inerten oder reduzierenden Atmosphäre erwärmt wird.

9. Verfahren nach Anspruch 3, wobei die Nadelrohlinge oder Nadel aus einer Wolframlegierung auf eine Temperatur zwischen 800 und 1150 °C in einer inerten oder reduzierenden Atmosphäre erwärmt wird bzw. werden.

10. Verfahren nach Anspruch 3, wobei die Nadelrohlinge oder Nadel aus einer Wolframlegierung auf eine Temperatur zwischen 350 und 900 °C in einer oxidierenden Atmosphäre erwärmt wird bzw. werden.

11. Gebogene Nähnadel, die mit dem Verfahren nach Anspruch 1 erhalten wird, wobei das Verfahren (1) ein Formen von Nadelrohlingen, die bis zu 30 Gewichtsprozent Rhenium und den Rest bis zu 100 Gewichtsprozent Wolfram umfassen, zu einer gebogenen Nähnadel; und (2) ein Erwärmen der gebogenen Nähnadel auf eine Temperatur zwischen 700 und 1900 °C in einer inerten oder reduzierenden Atmosphäre über eine Dauer von 0,01 bis 1 Stunde umfasst.

12. Nähnadel nach Anspruch 11, wobei das Verfahren ferner den Schritt des Erwärmens der Nadel auf eine Temperatur zwischen 350 und 900 °C in einer oxidierenden Atmosphäre über eine Dauer von 0,01 bis 1 Stunde umfasst.

13. Gebogene Nähnadel, die mit dem Verfahren nach Anspruch 1 erhalten wird, wobei das Verfahren ein Erwärmen von gebogenen chirurgischen Rohlingen aus einer Wolframlegierung oder von einer gebogenen Nadel auf eine Temperatur zwischen 350 und 900 °C in einer oxidierenden Atmosphäre über eine Dauer von 0,01 bis 1 Stunde umfasst.

## Revendications

1. Procédé de fabrication d'une aiguille à suture en alliage de tungstène courbe comportant les étapes consistant à chauffer des ébauches d'aiguille en alliage de tungstène courbe ou une aiguille à suture en alliage de tungstène courbe à une température dans la plage de 350 °C à une température inférieure à la température de recristallisation de l'alliage.

2. Procédé selon la revendication 1, dans lequel les ébauches d'aiguille en alliage de tungstène ou l'aiguille sont chauffées à une température dans la plage de 350 à 1 900 °C.

3. Procédé selon la revendication 2, dans lequel les ébauches d'aiguille en alliage de tungstène ou l'aiguille comprennent un ou plusieurs métaux choisis dans le groupe constitué par le rhénium, le tantale ou le molybdène.

4. Procédé selon la revendication 3, dans lequel les ébauches d'aiguille en alliage de tungstène ou l'aiguille comprennent du rhénium.

5. Procédé selon la revendication 2, dans lequel les ébauches d'aiguille en alliage de tungstène ou l'aiguille comprennent jusqu'à 30 pour cent en poids de rhénium, le complément étant du tungstène.

6. Procédé selon la revendication 5, dans lequel les ébauches d'aiguille en alliage de tungstène ou l'aiguille comprennent de 20 à 26 pour cent en poids de rhénium, le complément étant du tungstène.

7. Procédé selon la revendication 6, dans lequel les ébauches d'aiguille en alliage de tungstène ou l'aiguille sont chauffées pendant 0,5 heure entre 800 et 1 150 °C.

8. Procédé selon la revendication 1, dans lequel l'aiguille à suture courbe est chauffée à une température dans la plage de 700 à 1 900 °C dans une atmosphère inerte ou réductrice.

9. Procédé selon la revendication 3, dans lequel les ébauches d'aiguille en alliage de tungstène ou l'aiguille sont chauffées à une température dans la plage de 800 à 1 150 °C dans une atmosphère inerte ou réductrice.

10. Procédé selon la revendication 3, dans lequel les ébauches d'aiguille en alliage de tungstène ou l'aiguille sont chauffées à une température dans la plage de 350 à 900 °C dans une atmosphère oxydante.

11. Aiguille à suture courbe obtenue par le procédé selon la revendication 1, ledit procédé consistant à
(1) former des ébauches d'aiguille comprenant jusqu'à 30 pour cent en poids de rhénium, le complément étant du tungstène, en une aiguille à suture courbe ; et
(2) chauffer ladite aiguille à suture courbe à une température dans la plage de 700 à 1 900 °C dans une atmosphère inerte ou réductrice pendant 0,01 à 1 heure.

12. Aiguille à suture selon la revendication 11, le procédé comportant en outre l'étape consistant à chauffer ladite aiguille à une température dans la plage de 350 à 900 °C dans une atmosphère oxydante pendant 0,01 à 1 heure.

13. Aiguille à suture courbe obtenue par le procédé selon la revendication 1, ledit procédé consistant à chauffer des ébauches chirurgicales en alliage de tungstène courbes ou une aiguille courbe à une température dans la plage de 350 à 900 °C dans une atmosphère oxydante pendant 0,01 à 1 heure.
